# DEMANDE DE BREVET EUROPEEN

(11) **EP 4 613 104 A1**
(43) Date de publication de la demande: **10.09.2025**
(21) Numéro de dépôt: 25161206.5
(22) Date de dépôt: 03.03.2025
(51) Int. Cl.: A23J 1/04, A23J 1/10, A23K 10/20, A23K 10/22, A23L 33/18, A61K 35/60, A61P 29/00

(54) **HYDROLYSAT DE PROTEINES DE POISSONS ET APPLICATIONS CHEZ LES ANIMAUX D`ELEVAGE**

(30) Priorité: 05.03.2024 FR 2402219
(71) Demandeur: Agromousquetaires, 75015 Paris (FR)
(72) Inventeur: DRIEU LA ROCHELLE, Hubert, 75015 PARIS (FR)
(74) Mandataire: Cabinet Le Guen Maillet

(57) **Abrégé**

La présente invention concerne un hydrolysat de protéines provenant de tissus ou cartilages de poissons riches en en protéines, en particulier en collagène, et ses applications dans l'alimentation et le bien-être des animaux d'élevage, en particulier les animaux porcins et les volailles.

En particulier, l'invention concerne un hydrolysat obtenu par digestion enzymatique à l'aide d'une enzyme endopeptidase, ledit hydrolysat étant caractérisé en ce qu'il présente une fraction protéique présentant un profil moléculaire de répartition suivante :
- entre 15 et 60 % de peptides de poids moléculaire compris entre 1000 et 5000 Da,
- entre 20 et 40% de peptides de poids moléculaire compris entre 500 et 1000 Da,
- entre 10 et 40% de peptides de poids moléculaire compris entre 150 et 500 Da,
- entre 1 et 10% de peptides de poids moléculaire inférieur à 150 Da.

## Description

### DOMAINE TECHNIQUE

La présente invention se situe dans le domaine du bien-être animal et concerne un hydrolysat de protéines provenant de tissus ou cartilages de poissons riches en protéines, en particulier en collagène, ainsi qu'un procédé pour son obtention. L'hydrolysat selon l'invention trouve application dans l'alimentation et le maintien de bonnes conditions physiques des animaux d'élevage, notamment des animaux porcins et des volailles. En particulier, l'hydrolysat présente des effets antiinflammatoires et antioxydants limitant ou inhibant les phénomènes de douleur et les pertes de fonctions physiques, prévient ou traite les phénomènes de bursites et permet le maintien de l'animal dans des conditions favorisant ou stimulant la croissance de l'animal.

### ÉTAT DE LA TECHNIQUE ANTÉRIEUR

Le cycle de croissance du porc se divise généralement en plusieurs étapes, de la naissance à l'abattage.

Les porcelets naissent après une période de gestation d'environ 114 jours. Les porcelets pèsent entre 1,2 et 1,5 kg à la naissance. Ils restent avec leur mère durant trois à quatre semaines environ, et sont nourris par allaitement.

L'étape d'après est une la phase dite de post-sevrage durant six à huit semaines, environ. Les porcelets sont séparés de leur mère et réunis en enclos adaptés, c'est le sevrage. Ils passent ainsi d'une alimentation lactée à une alimentation végétale sous forme solide.

S'en suit l'étape d'engraissement durant trois à quatre mois. A l'issue de cette période, le porc, appelé porc charcutier, part à l'abattoir au poids vif d'environ 120 kg.

Durant les étapes de post-sevrage et d'engraissement, des problèmes de croissances peuvent apparaitre nuisant ainsi à la performance de l'animal. Les porcelets se trouvent en effet soumis à différentes sources de stress, tels que le stress alimentaire lié au changement d'alimentation, le stress environnemental lié au changement de logement et le stress social lié à leur séparation d'avec leur mère. On observe ainsi durant cette période des réactions de stress oxydatif et des réactions inflammatoires, souvent liées aux stress oxydatifs.

Un tel état nuit à la croissance de l'animal, c'est-à-dire à son gain de poids. On observe également les mêmes phénomènes chez d'autres animaux d'élevage, tels que les volailles

Un autre facteur qui nuit à la croissance des porcelets est l'apparitions de bursites. La bursite est due à une réaction inflammatoire de la bourse séreuse, une petite poche située près des articulations qui contient une petite quantité de liquide qui facilite le mouvement des tendons et des muscles sur les os adjacents. Lorsque cette bourse s'enflamme, on parle de bursite.

La bursite peut être causée par des blessures, des traumatismes ou des irritations. Les états favorisants le stress oxydatif et l'inflammation sont également sans doute des facteurs aggravants. Chez les porcs, elle apparait généralement sur la partie basse des membres postérieurs, au niveau des tarses et métatarses. Les bursites sont très visibles, elles provoquent un gonflement, une sensibilité, une rougeur et éventuellement une boiterie. Le traitement dépend de la cause sous-jacente de la bursite et peut inclure des mesures telles que l'administration d'anti-inflammatoires.

Les solutions aujourd'hui proposées pour améliorer le bien-être des animaux d'élevage, tels que les porcins et les volailles, se tournent vers des approches naturelles. Par exemple l'apport de vitamine E est proposé pour lutter contre le stress oxydatif. Plusieurs polyphénols, tels que le resvératrol, les catéchines, la quercétine ou la curcumine, sont également envisagés pour réduire le stress oxydatif. Cependant ils ne semblent pas efficaces pour traiter les réactions inflammatoires. Concernant les bursites des porcs, les traitements aujourd'hui proposés reposent uniquement sur la prise de médicaments anti-inflammatoires, l'application de crèmes antiseptiques ou sur l'amélioration de la qualité des sols des enclos.

Comme bien souvent, l'approche la plus satisfaisante est la prévention. Elle doit viser à la restauration d'une croissance normale et du bien-être de l'animal. La maîtrise des facteurs de risques en élevage, notamment de la qualité du sol, est un préalable indispensable mais malheureusement souvent insuffisant. En complément, une action sur les mécanismes responsables d'inflammations et de stress oxydatif est souhaitable afin d'atteindre l'obj ectif ci-dessus.

### EXPOSÉ DE L'INVENTION

Le but de la présente invention est ainsi de proposer une solution naturelle et efficace pour le bien-être des animaux d'élevage, tels que les porcins et les volailles, en particulier pour prévenir et traiter les réactions de stress oxydatifs et d'inflammations et limiter ou traiter les phénomènes de bursites, et favoriser la prise de poids et la croissance de l'animal.

A cet effet, l'invention concerne un hydrolysat de protéines de tissus ou de cartilages riches en collagène de poisson, obtenu par digestion enzymatique à l'aide d'une enzyme endopeptidase d'origine bactérienne, ledit hydrolysat étant caractérisé en ce qu'il présente une fraction protéique présentant un profil moléculaire de répartition suivante :
- entre 15 et 60 % de peptides de poids moléculaire compris entre 1000 et 5000 Da,
- entre 20 et 40% de peptides de poids moléculaire compris entre 500 et 1000 Da,
- entre 10 et 40% de peptides de poids moléculaire compris entre 150 et 500 Da,
- entre 1 et 10% de peptides de poids moléculaire inférieur à 150 Da,
et un aminogramme dans lequel, en pourcentage de la matière azotée totale :
- la teneur en acide glutamique est comprise entre 10 et 14%
- la teneur en valine est comprise entre 1,5 et 3,5%
- la teneur en isoleucine est comprise entre 1 et 3%
- la teneur en leucine est comprise entre 2 et 6%
- la teneur en glycine est comprise entre 9 et 13%
- la teneur en lysine est comprise entre 0,5 et 3%
- la teneur en arginine est comprise entre 6 et 10%.

L'hydrolysat selon l'invention est un mélange de peptides et acides aminés libres obtenu après hydrolyse enzymatique, à l'aide d'une enzyme spécifique, de matières premières provenant de poissons, telles que les peaux de poisson et les cartilages. Ces matières étant riches en collagène, les peptides et acides aminés libres composant l'hydrolysat sont principalement issu de collagène.

L'hydrolysat selon l'invention se montre particulièrement efficace pour stimuler la croissance des animaux d'élevage, comme le montrent les exemples qui suivent.

On notera d'ailleurs que l'hydrolysat selon l'invention est avantageusement riche en acides aminés précurseurs de l'hormone de croissance tels que l'arginine, la valine, l'acide glutamique, la lysine et les acides aminés à longue chaine BCAA c'est-à-dire la leucine, l'isoleucine et la valine. L'hormone de croissance augmente le volume musculaire, accroit la densité osseuse et diminue la synthèse de graisse.

De plus, l'hydrolysat selon l'invention se montre efficace pour prévenir et traiter les phénomènes de bursites chez les porcins, prévenir et traiter les réactions de stress oxydatif, d'inflammation, responsables des problèmes de croissance chez les animaux d'élevage.

L'effet antioxydant lutte contre le vieillissement cellulaire.

L'effet anti inflammatoire protège contre les inflammations, telles que les rougeurs, les enflures, la douleur et les pertes de fonctions physiques normales.

En conséquence, l'animal traité par un hydrolysat selon l'invention est un animal en bonne santé et qui peut donc poursuivre une croissance normale.

Avantageusement, l'hydrolysat selon l'invention présente, de plus :
- une teneur en matière grasse inférieure à 0,5 % en pourcentage d'extrait sec,
- une teneur en matière azotée totale (N * 6,25) supérieure à 90 %, en pourcentage d'extrait sec,
- une teneur en matière minérale inférieure à 10 %, en pourcentage d'extrait sec.

Préférentiellement, l'aminogramme présente, de plus, en pourcentage de la matière azotée totale :
- une teneur en acide aspartique est comprise entre 7 et 10%
- une teneur en proline est comprise entre 7 et 11%
- une teneur en thréonine est comprise entre 2 et 5%
- une teneur en sérine est comprise entre 3 et 7%
- une teneur en alanine est comprise entre 8 et 12%
- une teneur en cystine est comprise entre 0,1 et 1%
- une teneur en méthionine est comprise entre 1,5 et 3%
- une teneur en tyrosine est comprise entre 0,5 et 2 ,5%
- une teneur en phénylalanine est comprise entre 1 et 3,5%
- une teneur en histidine est comprise entre 3,5 et 7%
- une teneur en hydroxyproline est comprise entre 4 et 8%
- une teneur en hydroxylysine est comprise entre 0,5 et 2,5%

Préférentiellement, le tissu riche en collagène de poisson est de la peau.

Préférentiellement encore le poisson est un Salmonidae, préférentiellement du saumon ou de la truite, ou encore un poisson blanc.

Selon une caractéristique de l'invention, l'enzyme est une endoprotéase d'origine bactérienne commercialisée sous la marque Corolase 7089 par la société AB enzymes.

L'invention concerne encore un procédé d'obtention d'un hydrolysat de protéines de poissons tel que décrit précédemment, le procédé étant caractérisé en ce qu'il comprend les étapes de :
- préparation d'une source de protéines à partir de tissus et/ou cartilages de poisson riches en collagène,
- préparation d'une solution enzymatique comprenant entre 0,1 et 0.5% d'une enzyme endoprotéase,
- hydrolyse enzymatique de ladite source de protéines à une température comprise entre 40 et 60°C, pendant 1 à 5 heures, après l'ajout de la solution enzymatique,
- arrêt de l'hydrolyse enzymatique par chauffage à une température d'au moins 90°C durant 8 à 20 minutes,
- séparation de l'hydrolysat de protéines des autres composants,
- récupération et concentration de l'hydrolysat de protéines.

Selon le procédé de l'invention, l'enzyme est une endoprotéase d'origine bactérienne commercialisée sous la marque Corolase 7089 commercialisée par la société AB enzymes. Avantageusement, le rapport source de protéines/eau est inférieur ou égal à 1.

Préférentiellement, le tissu est de la peau.

Préférentiellement, le poisson est un Salmonidae, préférentiellement du saumon ou de la truite, ou encore un poisson blanc.

L'invention concerne encore une composition comprenant un hydrolysat de protéines tel que défini précédemment, ou obtenu par un procédé tel que défini précédemment.

Une composition selon l'invention peut se présenter sous toute forme connue et adaptée à l'alimentation des animaux d'élevage, telle qu'une solution, une composition solide telle qu'un comprimé, une poudre ou une gélule, un granule ou encore un gel. La composition peut comprendre tout excipient adapté, et tout autre composé tel que des vitamines, des minéraux, des actifs, des conservateurs, des colorants.

L'invention concerne encore un hydrolysat de protéines de tissus ou de cartilages riches en collagène de poisson tel que défini précédemment, ou obtenu par un procédé tel que défini précédemment, ou une composition en contenant, pour son utilisation dans l'alimentation des animaux d'élevage, en particulier les animaux porcins et les volailles.

L'invention concerne encore un hydrolysat de protéines de tissus ou de cartilages riches en collagène de poisson tel que défini précédemment, ou obtenu par un procédé tel que défini précédemment, ou une composition en contenant, pour la stimulation de la croissance des animaux d'élevage, en particulier les animaux porcins et les volailles.

Avantageusement, la stimulation de la croissance comprend un gain de poids, en particulier un gain de muscles.

L'invention concerne encore un hydrolysat de protéines de tissus ou de cartilages riches en collagène de poisson tel que défini précédemment, ou obtenu par un procédé tel que défini précédemment, ou une composition en contenant, pour la prévention et le traitement des bursites.

L'invention concerne encore un hydrolysat de protéines de tissus ou de cartilages riches en collagène de poisson tel que défini précédemment, ou obtenu par un procédé tel que défini précédemment, ou une composition en contenant, pour la prévention et le traitement des réactions antioxydantes et antiinflammatoires chez les animaux d'élevage, en particulier les animaux porcins et les volailles.

Dans le cadre de l'invention, les animaux peuvent être des animaux en bonne santé ou souffrant d'un retard de croissance. En effet, le traitement des animaux à l'aide d'un hydrolysat selon l'invention peut être préventif ou curatif.

Avantageusement, l'hydrolysat, ou la composition en contenant, sont administrés aux animaux porcins durant la période d'engraissement, préférentiellement à partir de la 6eme semaine après la naissance, durant 6 semaines, ou durant les trois à six dernières semaines de la période d'engraissement.

Préférentiellement encore, dans le cadre de l'invention, l'hydrolysat, ou la composition en contenant, sont administrés à raison de 3 à 7 g/jour d'hydrolysat. Ce dosage journalier convient particulièrement à un produit dont l'extrait sec est de 50%.

Partant du dosage journalier nécessaire, l'homme du métier saura formuler une composition adaptée à un tel dosage.

Les caractéristiques de l'invention mentionnées ci-dessus, ainsi que d'autres, apparaîtront plus clairement à la lecture des exemples qui suivent et en lien avec le dessin joint suivant :
[Fig. 1] est diagramme représentant la répartition du poids moléculaire d'hydrolysats de protéines selon l'invention,
[Fig. 2] est un diagramme représentant les moyennes de production d'interleukine 6, en pg/µg de protéines, après exposition de cellules humaines de kératinocytes aux UV-B, les cellules ayant été mises en culture avec un hydrolysat selon l'invention à différentes concentrations, ou avec de la vitamine D3, de l'éthanol, ou simplement un milieu de culture,
[Fig. 3] est un diagramme en courbe représentant les équivalent Trolox d'un hydrolysat selon l'invention en fonction du Net-AUC (Aire sous la courbe nette).

### EXEMPLES

### Exemple 1 : obtention d'hydrolysats de protéines de peaux de saumon

Quatre hydrolysats de protéines de peaux de saumon ont été préparés selon le procédé suivant :
- Mélange de peaux de saumon avec 1 volume d'eau et 0,2 % d'enzyme Corolase 7089^{®}
- Hydrolyse enzymatique durant 1 à 5 H à une température de 40 à 60°C,
- Inactivation de l'enzyme par chauffage à une température d'au moins 90°C durant 8 à 20 minutes,
- Tamisage sur tamis de 300 µm afin d'éliminer les résidus d'hydrolyse,
- Centrifugation du jus d'hydrolyse 10 à 15 min à 4700 tr/min pour séparer l'huile, les insolubles et l'hydrolysat de protéines,
- Concentration de l'hydrolysat.

Le Tableau 1 indique les caractéristiques des hydrolysats.

**[TAB. 1]**

| **Paramètres** | **Composition extrait sec** |
|---|---|
| Extrait sec (%) | 100 % |
| Protéines totales (N*6,25) | >90% |
| Matières grasses | < 0,5 % |
| Matières minérales | <10% |

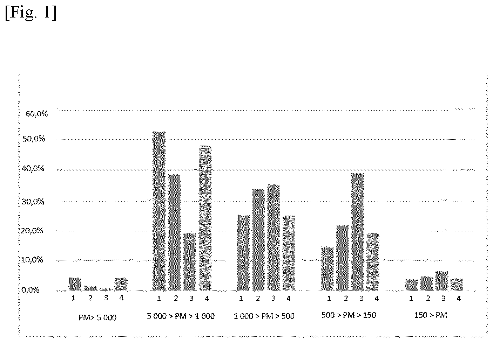

La Figure 1 indique la répartition des poids moléculaires des quatre hydrolysats.

### Exemple 2 : évaluation des propriétés anti-inflammatoires d'un hydrolysat selon l'invention

Le but de cet essai est d'évaluer l'activité anti-inflammatoire d'un hydrolysat obtenu par un procédé tel que celui décrit dans l'exemple 1 sur des cellules de kératinocytes humaines irradiées par exposition aux UV-B.

Dans cet exemple, des cellules de kératinocytes humaines sont pré-incubées avec les composés à tester durant 24h avant exposition aux UV-B. Les surnageants sont collectés 24h après traitement UV-B et la sécrétion d'interleukines 6 (IL-6) est quantifiée selon le test ELISA.

L'interleukine 6 joue un rôle de messager entre les cellules impliquées dans la régulation de l'inflammation aigüe et chronique. Une hyperproduction d'interleukine 6 provoque l'inflammation et peut être lié à l'origine de lésions articulaires.

### Contrôles témoins :

Témoin positif : La 1,25-dihydroxyvitamine D3 (0,24 µM diluée dans milieu de culture BSA) est utilisée comme en tant qu'inhibiteur connu de la sécrétion d'IL-6 (De HAES et al. (J. Cell. Biochem., 89(4):663-73, 2003)).

Témoin solvant : éthanol (0,1% dilué dans milieu de culture BSA) et milieu de culture.

### Cellules de kératinocytes : lignée cellulaire HEK001

### Protocole :

A D0, les cellules de kératinocyte sont réparties, dans leur milieu de cuture, dans des plaques 12 puits. Elles sont incubées à 37°C, 5% de CO2, pour obtenir une confluence inférieure à 50% à D1.

A D1, les cellules sont traitées avec les composés suivants :
- Hydrolysat selon l'invention selon cinq concentrations : 0,06 mg/ml ; 0,31 mg/ml ; 1,57 mg/ml ; 7,84 mg/ml ; 39,20 mg/ml
- 0.24 µM de1,25-dihydroxyvitamine D3
- 0,1% d'éthanol
- Milieu de culture

### Chaque traitement est testé avec et sans exposition aux UV-B.

### Traitement aux UV-B :

Les cellules sont irradiées à une dose de 12 mJ/cm² d'UVB à 312 nm.

Après irradiation, les cellules sont incubées dans du milieu de culture frais durant 24H à 37°C-5 % de CO₂.

### Analyses :

Après 24H d'incubation, les cellules sont lysées et traitées de manière à recueillir les fractions protéiques.

Les concentrations protéiques sont évaluées par le kit de dosage de protéines BCA selon la méthode standardisée (TEC-136).

La sécrétion de l'interleukine 6 est quantifiée par le test ELISA grâce au kit "Quantikine^{®} Human IL-6 Immunoassay". La concentration en IL-6 est exprimée en « pg/µg de protéines » sur la base de la quantité totale en protéines évaluées par le test BCA.

Les analyses sont répétées deux fois.

### Résultats :

La production d'IL-6 sans irradiations aux UV-B pour les contrôles a été dosée de manière à valider l'expérience. Les résultats sont indiqués dans le tableau 2.

**[TAB. 2]**

| **Composé** | **Rétro calcul IL-6 (pg/ml)** | **Moyenne** | **µg/ml protéines** | **pg IL-6 / µg proteins** | **Moyenne** |
|---|---|---|---|---|---|
| Milieu de culture | 9.87 | 9.49 +/-0,55 | 264.70 | 0.04 | 0.04 +/- 0,00 |
| | 9.10 | | 246.52 | 0.04 | |
| Ethanol | 9.41 | 7.32 +/- 2,96 | 208.62 | 0.05 | 0.05 +/-0,00 |
| | 5.23 | | 114.49 | 0.05 | |
| Vit D3 | 8.02 | 8.41 +/-0,55 | 198.00 | 0.04 | 0.04 +/-0,00 |
| | 8.79 | | 245.00 | 0.04 | |

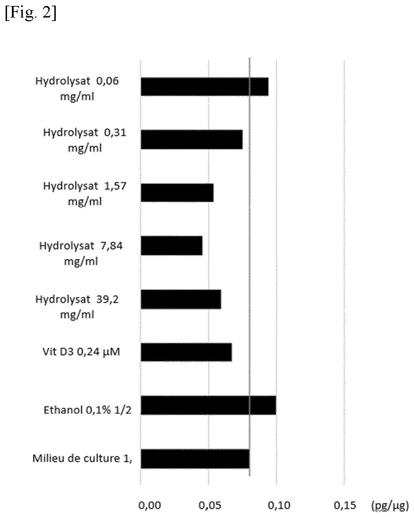

Les résultats des dosages de la production d'IL-6 avec irradiations aux UV-B sont présentés dans les tableaux 3 et 4, ainsi qu'à la figure 2.

**[TAB. 3]**

| **Echantillon** | **Rétro calcul IL-6 (pg/ml)** | **Moyenne** | **µg/ml protéines** | **pg IL-6 / µg protéines** | **Moyenne** |
|---|---|---|---|---|---|
| Milieu de culture | 21.85 | 20.63 +/-1,73 | 255.24 | 0.09 | 0.08 +/-0,01 |
| | 19.40 | | 263.72 | 0.07 | |
| Ethanol | 24.75 | 24.45 +/-0,43 | 243.93 | 0.10 | 0.10 +/-0,00 |
| | 24.14 | | 245.34 | 0.10 | |
| Vit D3 | 14.34 | 13.88 +/-0,65 | 201.42 | 0.07 | 0.07 +/-0,01 |
| | 13.42 | | 211.35 | 0.06 | |

**[TAB. 4]**

| **Echantillon** | **Rétro calcul IL-6 (pg/ml)** | **Moyenne** | **µg/ml protéines** | **pg IL-6 / µg protéines** | **Moyenne** |
|---|---|---|---|---|---|
| Hydrolysat 39.2 mg/ml | 11.73 | 11.11 +/- 0,87 | 202.84 | 0.06 | 0.06 +/-0.00 |
| | 10.49 | | 175.86 | 0.06 | |
| Hydrolysat 7.84 mg/ml | 12.19 | 11.88 +/-0,44 | 265.13 | 0.05 | 0.05 +/-0,00 |
| | 11.57 | | 255.24 | 0.05 | |
| Hydrolysat 1.57 mg/ml | 12.96 | 12.19 +/-1,09 | 269.37 | 0.05 | 0.05 +/-0,01 |
| | 11.42 | | 192.91 | 0.06 | |
| Hydrolysat 0.31 mg/ml | 14.03 | 16.87 +/-4,01 | 217.02 | 0.06 | 0.07 +/-0,01 |
| | 19.71 | | 231.19 | 0.09 | |
| Hydrolysat 0.06 mg/ml | 19.25 | 23.30 +/-5,72 | 243.93 | 0.08 | 0.09 +/-0,02 |
| | 27.34 | | 249.59 | 0.11 | |

### Interprétation :

On note que le traitement aux UV-B induit bien une augmentation de la production d'IL-6 (Tableau 2 versus Tableau 3).

Le traitement avec la vitamine D3 diminue bien la production d'IL-6, en comparaison avec le traitement par du milieu de culture qui sert de référence: 13,8 +/- 0,65 comparé à 20,63 +/-1,73 pg/ml, et 0,07 +/- 0,01 comparé à 0,08 +/- 0,01 pg/µg ( Voir le Tableau 3, et la figure 2 sur laquelle le trait indique la référence, c'est-à-dire le niveau de production d'IL-6 en conditions de contrôle). La vitamine D3 est donc bien un témoin positif.

L'hydrolysat selon l'invention induit une diminution de la production d'IL-6 aux concentrations de 39,2 mg/ml (0,06 +/- 0,00 pg/µg comparé à 0,08 +/- 0,01 pg/µg), 7,84 mg/ml (0,05 +/- 0,00 pg/µg comparé à 0,08 +/- 0,01 pg/µg), 1,57 mg/ml (0,05 +/- 0,01 pg/µg comparé à 0,08 +/- 0,01 pg/µg) et 0,31 mg/ml (0,07 +/- 0,01 pg/µg comparé à 0,08 +/- 0,01 pg/µg) (Voir le Tableau 4 et la figure 2).

Par ailleurs l'hydrolysat selon l'invention induit une diminution de la production d'IL-6 supérieure à celle du témoin positif aux concentrations de 39,2 mg/ml (0,06 +/- 0,00 pg/µg comparé à 0,07 +/- 0,01 pg/µg), 7,84 mg/ml (0,05 +/- 0,00 pg/µg comparé à 0,07 +/- 0,01 pg/µg), 1,57 mg/ml (0,05 +/- 0,01 pg/µg comparé à 0,07 +/-0,01 pg/µg) (Voir le Tableau 4 et la figure 2).

L'hydrolysat selon l'invention présente bien une activité anti-inflammatoire.

### Exemple 3 : évaluation des propriétés anti-oxydantes d'un hydrolysat selon l'invention

Le but de cet essai est d'évaluer les propriétés antioxydantes d'un hydrolysat selon l'invention conformément à la méthode ORAC ((acronyme anglais pour Oxygen Radical Absorbance Capacity signifiant en français capacité d'absorption des radicaux oxygénés).

L'hydrolysat testé est obtenu selon un procédé tel que décrit dans l'exemple 1.

L'hydrolysat est testé selon les concentrations suivantes : 20 mg/ml; 10 mg/ml; 2 mg/ml; 1 mg/ml; 0,200 mg/ml; 0,100 mg/ml; 0,020 mg/ml; 0,010 mg/ml; 0,002 mg/ml and 0,001 mg/ml (solvant tampon phosphate 75 mM pH 7,4).

Contrôle témoin positif : acide 3,4-dihydro-6-hydroxy-2,5,7,8-tétraméthyl-2H-1-benzopyran-2-carboxylique (Trolox). Le Trolox est préparé à 2 mM (0,5 mg/ml) dans 75 mM de tampon phosphate pH 7,4 et dilué dans le même tampon aux concentrations: 1,0000 mM, 0,5000 mM, 0,2500 mM, 0,1250 mM et 0,0625mM. 8 µl de chaque solution sont ajoutés dans chaque puits afin d'obtenir les concentrations tests de 50 µM, 25 µM, 12,500 µM, 6,250 µM et 3,125 µM (160 µl de volume final).

### Réactifs :

- Tampon KPO4, pH 7,4, à 75 mM.
- 2,2'-Azobis(2-méthylpropionamidine) dihydrochloride (AAPH) à 70 mM (18,98 mg/ml) dans 75 mM de tampon phosphate, pH 7,4. 16 µL sont ajoutés dans chaque puits pour obtenir les concentrations tests (volume final de 160 µL). l'AAPH est utilisé comme source de radical péroxyde.
- Fluorescéine (FL) préparée à 2,66 mM (1 mg/ml) dans 75 mM de tampn phosphate, pH 7,4, puis diluée dans le même tampon jusqu'à une concentration de 0,8235 nM. 136 µl sont ajoutés dans chaque puits pour obtenir un concentration test de 0,7 nM (volume final de 160 µL).

### Protocole

L'évaluation ORAC a été réalisée conformément à la méthode standard (TEC-151). 136 µl de 0,8235 fluorescéine et 8 µl d'échantillon d'hydrolysat, de solution Trolox (contrôle positif), ou 75 mM de tampon phosphate (contrôle négatif) ont été ajoutés dans les puits appropriés. Un contrôle supplémentaire, sans AAPH, contenant 136 µl de 0,8235 fluorescéine et 24 µl de tampon phosphate a été réalisé. Chaque condition est dupliquée.

Les plaques de puits ont été couvertes et incubées à 37 °C durant 15 min.

Les réactions ont été initiées par ajout de 16 µl de AAPH à 70 mM, à l'exception des puits sans AAPH. Les plaques ont été couvertes et incubées à 37 °C durant 60 min. La fluorescence a été mesurée toutes les minutes (excitation 450-490 nm, émission 510 et 530 nm) durant l'incubation.

### Détermination des valeurs ORAC

La consommation de la fluorescéine associée à son incubation en présence de AAPH a été estimée par mesure de la fluorescence. Une courbe typique du suivi de fluorescence au cours du test ORAC a été réalisée. L'aire sous la courbe nette (Net-AUC) (AUC avec hydrolysat - AUC sans hydrolysat) est calculée et le résultat est standardisé par rapport au trolox.

### Résultats

Le Tableau 5 illustre les valeurs Net-AUC pour le Trolox en fonction des doses indiquées.

**[TAB. 5]**

| **Trolox** | |
|---|---|
| **Dose (µM)** | **Net AUC** |
| 25.000 | 21.281 |
| 12.500 | 13.963 |
| 6.250 | 9.302 |
| 3.125 | 4.405 |
| 0.000 | 0.000 |

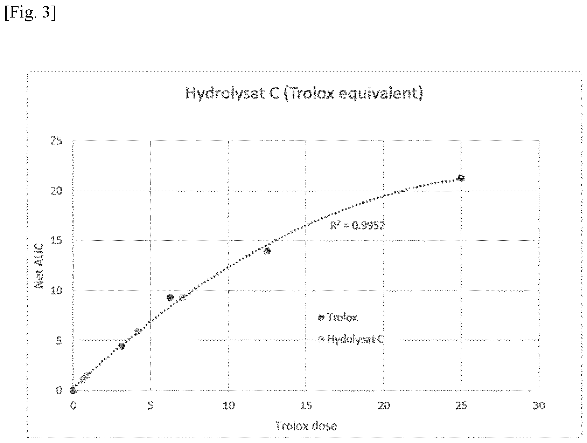

Quatre doses testées de l'hydrolysat selon l'invention présentent des valeurs Net-AUC dans la gamme des valeurs Net-AUC du trolox et permettent le calcul d'équivalents Trolox (Fig. 3 Tableau 5) :
- la dose de 0,02 mg/ml équivaut à 7,049 µM de Trolox
- la dose de 0,01 mg/ml équivaut à 4,150 µM de Trolox
- la dose de 0,002 mg/ml équivaut à 0,896 µM de Trolox
- la dose de 0,001 mg/ml équivaut à 0,571 µM de Trolox

L'extrapolation des données pour 1 g et 100 g d'hydrolysat est également présentée dans le Tableau 6. L'hydrolysat de protéines présente en moyenne un équivalent Trolox de 279 145 180 +/- 57 535 342 µM d'équivalent Trolox pour 100 g de produit. A titre de comparaison, le clou de girofle, l'un des aliments présentant les plus fortes activités antioxydantes, présente un équivalent Trolox de 314 446 µM d'équivalent Trolox pour 100 g de produit. Ainsi, l'hydrolysat de protéines selon l'invention présente une activité antioxydante environ 800 fois supérieure à celle du clou de girofle.

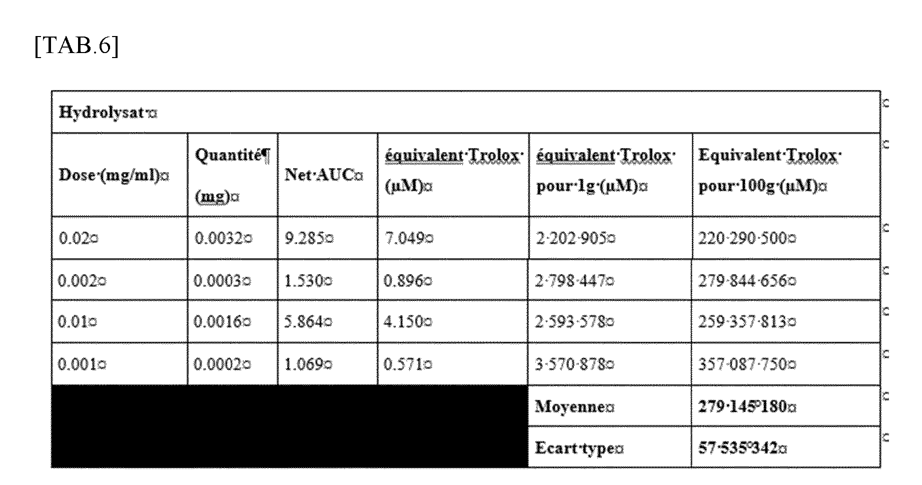

### Exemple 4 : Effets sur la croissance des porcs d'un hydrolysat selon l'invention

Au début de l'étude, les porcs sont âgés de 8 semaines environ et sont entrés dans la période d'engraissement qui s'étale ici sur 15 semaines, jusqu'à l'abattage.

Les porcs sont répartis en plusieurs lots :
Lot 1 : porcs ayant reçu l'hydrolysat selon l'invention durant les 2 semaines précédant l'abattage, à raison de 5g/jour.
Lot 2 : porcs ayant reçu l'hydrolysat selon l'invention durant les 3 semaines précédant l'abattage, à raison de 5g/jour.
Lot 3 : porcs ayant reçu l'hydrolysat selon l'invention durant les 6 semaines précédant l'abattage, à raison de 5g/jour.
Lot 4 : porcs ayant reçu l'hydrolysat selon l'invention durant 6 semaines en milieu de la période d'engraissement, à raison de 5g/jour.
Lot 5 : porcs ayant reçu l'hydrolysat selon l'invention durant 6 semaines à partir du début de la période d'engraissement, à raison de 5g/jour.

Pour chaque lot, des témoins ont été suivis, qui n'ont pas reçu l'hydrolysat selon l'invention.

Les animaux sont pesés au début et à la fin de la période d'engraissement. Les résultats sont présentés dans le tableau 7. Dans ce tableau sont indiqués pour chaque lot les gains moyens en poids quotidien (« GMQ) pour les porcs ayant reçu l'hydrolysat (« Traités ») et les porcs n'ayant pas reçus l'hydrolysat (« Témoins »). Les écarts entre les Traités et les Témoins pour chaque lot sont indiqués.

**[TAB. 7]**

| | | | Nombre | Poids initial (kg) | GMQ (g/j) | Écart (g) |
|---|---|---|---|---|---|---|
| Lot 1 | 2 sem. fin | Témoins | 78 | 20,7 | 754 | + 37 |
| | | Traités | 122 | 22,2 | 791 | |
| Lot 2 | 3 sem. fin | Témoins | 225 | 21,9 | 782 | + 57 |
| | | Traités | 185 | 22,8 | 839 | |
| Lot 3 | 6 sem. fin | Témoins | 145 | 25,9 | 752 | + 74 |
| | | Traités | 157 | 27,5 | 826 | |
| Lot 4 | 6 sem. milieu | Témoins | 163 | 24,8 | 750 | + 40 |
| | | Traités | 158 | 24,8 | 790 | |
| Lot 5 | 6 sem. début | Témoins | 176 | 22,9 | 768 | +5 |
| | | Traités | 125 | 25,0 | 773 | |

Un gain de pois quotidien est observé chez les animaux ayant reçu l'hydrolysat pour l'ensemble des lots. On relève toutefois que le gain de poids quotidien augmente lorsque l'hydrolysat est distribué en fin de période d'engraissement : + 57g/j par rapport aux témoins lorsqu'il est distribué les 3 dernières semaines (lot 2) et +74g/j par rapport aux témoins lorsqu'il est distribué les 6 dernières semaines (lot 3). Ala fin de la période d'engraissement les porcs du lot 2 pèsent en moyenne 4 kg de plus que les témoins et les porcs du lot 3 pèsent en moyenne 5 kg de plus que les témoins.

## Revendications

1. Hydrolysat de protéines de tissus ou de cartilages riches en collagène de poisson, obtenu par digestion enzymatique à l'aide d'une enzyme endopeptidase d'origine bactérienne, ledit hydrolysat étant **caractérisé en ce qu'**il présente une fraction protéique présentant un profil moléculaire de répartition suivante :
- entre 15 et 60 % de peptides de poids moléculaire compris entre 1000 et 5000 Da,
- entre 20 et 40% de peptides de poids moléculaire compris entre 500 et 1000 Da,
- entre 10 et 40% de peptides de poids moléculaire compris entre 150 et 500 Da,
- entre 1 et 10% de peptides de poids moléculaire inférieur à 150 Da,
et un aminogramme dans lequel, en pourcentage de la matière azotée totale :
- la teneur en acide glutamique est comprise entre 10 et 14%,
- la teneur en valine est comprise entre 1,5 et 3,5%
- la teneur en isoleucine est comprise entre 1 et 3%,
- la teneur en leucine est comprise entre 2 et 6%,
- la teneur en glycine est comprise entre 9 et 13%,
- la teneur en lysine est comprise entre 0,5 et 3%,
- la teneur en arginine est comprise entre 6 et 10%.

2. Hydrolysat selon la revendication 1, **caractérisé en ce qu'**il présente, de plus :
- une teneur en matière grasse inférieure à 0,5 % en pourcentage d'extrait sec,
- une teneur en matière azotée totale (N * 6,25) supérieure à 90 %, en pourcentage d'extrait sec,
- une teneur en matière minérale inférieure à 10 %, en pourcentage d'extrait sec.

3. Hydrolysat selon la revendication 1 ou 2, **caractérisé en ce que** l'aminogramme présente, de plus, en pourcentage de la matière azotée totale :
- une teneur en acide aspartique est comprise entre 7 et 10%
- une teneur en proline est comprise entre 7 et 11%
- une teneur en thréonine est comprise entre 2 et 5%
- une teneur en sérine est comprise entre 3 et 7%
- une teneur en alanine est comprise entre 8 et 12%
- une teneur en cystine est comprise entre 0,1 et 1%
- une teneur en méthionine est comprise entre 1,5 et 3%
- une teneur en tyrosine est comprise entre 0,5 et 2 ,5%
- une teneur en phénylalanine est comprise entre 1 et 3,5%
- une teneur en histidine est comprise entre 3,5 et 7%
- une teneur en hydroxyproline est comprise entre 4 et 8%
- une teneur en hydroxylysine est comprise entre 0,5 et 2,5%.

4. Hydrolysat selon l'une des revendications précédentes, caractérisé en ce ledit tissu est de la peau.

5. Hydrolysat selon l'une des revendications précédentes, caractérisé en ce ledit poisson est un Salmonidae, préférentiellement du saumon ou de la truite, ou un poisson blanc.

6. Hydrolysat selon l'une des revendications précédentes, caractérisé en ce ladite enzyme est l'enzyme commercialisée sous la marque Corolase 7089.

7. Procédé d'obtention d'un hydrolysat de protéines de poissons tel que défini dans l'une des revendications 1 à 6, **caractérisé en ce qu'**il comprend les étapes de :
- préparation d'une source de protéines à partir de tissus et/ou cartilages de poisson riches en collagène,
- préparation d'une solution enzymatique comprenant entre 0,1 et 0.5% d'une enzyme endopeptidase,
- hydrolyse enzymatique de ladite source de protéines à une température comprise entre 40 et 60°C, pendant 1 à 5 heures, après l'ajout de la solution enzymatique,
- arrêt de l'hydrolyse enzymatique par chauffage à une température d'au moins 90°C durant 8 à 20 minutes,
- séparation de l'hydrolysat de protéines des autres composants,
- récupération et concentration de l'hydrolysat de protéines.

8. Procédé selon la revendication 7, **caractérisé en ce que** ladite enzyme est l'enzyme commercialisée sous la marque Corolase 7089.

9. Procédé selon la revendication 7 à 8, **caractérisé en ce que** le rapport source de protéines/eau est inférieur ou égal à 1.

10. Procédé selon l'une des revendications 7 à 9, **caractérisé en ce que** ledit tissu est de la peau.

11. Procédé selon l'une des revendications 7 à 10, caractérisé en ce ledit poisson est un Salmonidae, préférentiellement du saumon ou de la truite, ou encore un poisson blanc.

12. Composition **caractérisée en ce qu'**elle comprend un hydrolysat de protéines tels que défini dans l'une des revendications 1 à 6, ou obtenu par un procédé tel que défini dans l'une des revendications 7 à 11.

13. Hydrolysat de protéines de tissus ou de cartilages riches en collagène de poisson tel que défini dans l'une des revendications 1 à 6, ou obtenu par un procédé tel que défini dans l'une des revendications 7 à 11, ou composition en contenant, pour la stimulation de la croissance des animaux d'élevage, en particulier les animaux porcins et les volailles.

14. Hydrolysat selon la revendication 13, caractérisé en ce la stimulation de la croissance comprend un gain de poids, en particulier un gain de muscles.

15. Hydrolysat de protéines de tissus ou de cartilages riches en collagène de poisson tel que défini dans l'une des revendications 1 à 6, ou obtenu par un procédé tel que défini dans l'une des revendications 7 à 11, ou composition en contenant, pour la prévention et le traitement des bursites.

16. Hydrolysat de protéines de tissus ou de cartilages riches en collagène de poisson tel que défini dans l'une des revendications 1 à 6, ou obtenu par un procédé tel que défini dans l'une des revendications 7 à 11, ou composition en contenant, pour la prévention et le traitement des réactions d'oxydations et des réactions inflammatoires chez les animaux d'élevage, en particulier les animaux porcins et les volailles.

17. Hydrolysat de protéines de poisson ou composition en contenant selon l'une des revendications 13 à 16, **caractérisé en ce que** ledit hydrolysat, ou ladite composition, sont administrés aux animaux porcins durant la période d'engraissement, préférentiellement à partir de la 6^{eme} semaine après la naissance, durant 6 semaines, ou durant les trois à six dernières semaines de la période d'engraissement.

18. Hydrolysat de protéines de poisson ou composition en contenant selon l'une des revendications 13 à 16, **caractérisé en ce que** ledit hydrolysat, ou ladite composition, sont administrées à raison de 3 à 7 g/jour d'hydrolysat.
